# EUROPEAN PATENT APPLICATION

(11) **EP 3 266 300 A1**
(43) Date of publication of application: **10.01.2018**
(21) Application number: 16178242.0
(22) Date of filing: 06.07.2016
(51) Int. Cl.: A01H 5/02

(54) **RUFFLE ROSE HAVING IMPROVED LONGEVITY**

(71) Applicant: Interplant Roses B.V., 3481 JA Harmelen (NL)
(72) Inventor: ILSINK, Robert Louis, 3481 JA Harmelen (NL); ILSINK, Martijn Frederik, 3481 JA Harmelen (NL); VAN DOESUM, Adrianus Hendricus, 3481 JA Harmelen (NL)
(74) Representative: Algemeen Octrooi- en Merkenbureau B.V.

(57) **Abstract**

The invention relates to a cut rose, comprising at least one flower comprising a plurality of petals, each petal being connected to the stem at the basal spot and having a longitudinal axis departing radially from the basal spot, the circumference of at least one of the said petals describing at least two recesses at opposite sides of the longitudinal axis, said recesses being directed towards the interior of the petal over a length *l*, therewith defining a tongue shaped petal portion therebetween, extending along the longitudinal axis, and a width w defined by the distance between the said recesses measured perpendicular to the longitudinal axis, said width w being equal to or less than length *l*.

## Description

The invention relates to a ruffle rose having improved longevity.

Ruffle roses are known in the art. Ruffle roses are very popular because of the particular shape of the petals of the flowers, resulting in a very attractive flower form. The circumference of said petals describe, starting from the basal spot where the petal is connected to the stem, two or more inwardly directed recesses, giving the petal a ruffled character. However, ruffle roses known in the art are so-called garden roses, intended to grow outdoors, the plants being rooted in the soil. Garden roses, and therewith the ruffle roses known in the art, are not suitable to be kept in a vase indoors, and have a limited longevity when cut and kept on water. Known ruffle roses will wither within two days.

Herein, the terms 'ruffle rose' or 'rose of the ruffle type' are intended to encompass rose plants, capable of producing flowers comprising a plurality of petals, each petal being connected to the stem at the basal spot and having a longitudinal axis departing radially from the basal spot, the circumference of at least one of the said petals describing at least two recesses at opposite sides of the longitudinal axis, said recesses being directed towards the interior of the petal over a length *l*, therewith defining a tongue shaped petal portion therebetween, extending along the longitudinal axis, and a width w defined by the distance between the said recesses measured perpendicular to the longitudinal axis, said width w being equal to or less than length *l*, i.e. the inwardly extending recesses in the petal defining a tongue portion of an elongated shape. Examples of known ruffle roses are e.g. marketed by Interplant Roses, the Netherlands, such as Glamorous Ruffles, Golden Lady Ruffles, and Pink Lady Ruffles.

Roses that are suitable to be kept indoors in water when cut are known in the art, and known as so-called cut roses. Cut roses herein are defined as roses, stems of which comprising a rose flower staying fresh for at least 6 days when cut and kept in water at ambient temperature. With the term 'staying fresh' is meant that the stem and the rose flower do not significantly wither, without significant bending of the stem allowing the flower to hang downward. The turgor is not significantly affected by keeping the cut rose in water. Also, discoloration, deterioration or local necrosis of the petals during a 6 day period of being kept in water does not significantly take place. Examples of known cut roses are e.g. known from Interplant, the Netherlands, such as Explorer (Interonontov, EU34993), Moonwalk (Intergeklawoom, EU38085), Prosecco (Interocceso, EU43829), Malibu (Interulim, EU25431).

It is a desire in the art to develop ruffle roses that can be kept as cut rose indoor in a vase, with improved longevity, i.e. staying fresh for at least 6 days, preferably for at least 7, 8, 9 or 10 days. However, in the art it has not been possible to develop such roses by one or more crossings with a known garden rose of the ruffle type as first parental strain and a cut rose as second parental strain.

The present inventors however, succeeded in development of cut roses of the ruffle type by performing one or more crossings with a plurality of parental lines, a first parental line being a garden rose of the ruffle type, and a second parental line being a cut flower rose capable of providing a plurality of flowers per stem. Said second parental line is a so-called 'polyantha', i.e. providing more than one flower per stem. By introducing a crossing step with a cut rose of the polyantha type, cut roses of the ruffle type could surprisingly be developed. Without being bound to any explanation, the inventors believe that by crossing with a cut rose having more flowers per stem, more seeds would be generated to improve the number of offspring plants.

In view of the above, it was contemplated to improve the crossing even more by choosing the first parental line having the floribunda phenotype. The term 'floribunda' is known in the art and originate from crossing hybrid teas with polyantha roses, and has two to four flowers per stem. When a garden rose of the ruffle and floribunda type is used as first parental line, the germination even increases as both first and second parental lines are of the polyantha type, resulting in offspring with more flowers, and therewith more seed bottles as compared to single-flower roses, therewith improving the selection capability, enabling identification and selection of candidates for cut ruffle roses. To this end, the second parental line preferably has more than 4 flowers per stem, preferably more than 10. Such phenotype is also known as so-called 'spray phenotype'. Further, it was observed that germination of spray roses is better than of single flower roses, i.e. more seeds develop to a plantlet, which further enhances the development and number of offspring. It was observed that using a polyantha as parental line, preferably of the spray phenotype, in particular in combination with a parental line of the floribunda phenotype, a number of offspring above a critical value could be reached, enabling cut roses of the ruffle type to be identified or further developed and to be selected, e.g. for further breeding.

Although it is not necessary to cross the first parental line with the second parental line directly, such direct crossing is preferred. By direct crossing, less steps are to be taken for the development of cut ruffle roses. However, the first parental line can also be crossed with another line before the offspring is crossed with the second parental line or vice versa. It is important that the eventual offspring originates from both first and second parental line.

In a very attractive embodiment, a crossing with a third parental line, the third parental line being a cut flower rose providing a single flower per stem, i.e. of the single flower type, is performed after crossing with the first and the second parental line. By such a crossing, the polyantha phenotype can be removed, resulting in cut roses of the single flowered ruffle type.

The selection preferably comprises selecting offspring from one or more crossings on the presence of at least commencing ruffle phenotype in one or more flowers, i.e. said flowers having one or more petals, the circumference thereof describing at least two recesses at opposite sides of the longitudinal axis, said recesses being directed towards the interior of the petal. In particular preferably at least 10%, more preferably at least 20%, more preferably at least 30%, even more preferably at least 40% and most preferably at least 50% of the said petals describing at least two such recesses. Mostly preferred, the majority of the petals describe such recesses, in particular at least 60%, 70%, 80% or 90% of the petals. As soon as an offspring plant develops one or more flowers of such a phenotype, it can be further crossed to obtain the more pronounced ruffle phenotype, e.g. by crossing offspring that have, in a previous generation or generations, one parent in common, so-called half-sib crossings.

The selection preferably comprises selection of true ruffle phenotype as defined herein, i.e. selecting offspring from one or more crossings on the presence of one or more flowers comprising a plurality of petals, each petal being connected to the stem at the basal spot and having a longitudinal axis departing radially from the basal spot, the circumference of at least one, preferably at least 10%, more preferably at least 20%, more preferably at least 30%, even more preferably at least 40% and most preferably at least 50% of the said petals describing at least two recesses at opposite sides of the longitudinal axis, said recesses being directed towards the interior of the petal over a length *l*, therewith defining a tongue shaped petal portion therebetween, extending along the longitudinal axis, and a width w defined by the distance between the said recesses measured perpendicular to the longitudinal axis, said width w being equal to or less than length *l*. It advantageous to select offspring with pronounced ruffle phenotype, as it was found that less pronounced ruffle phenotype may tend to become lost after more periods of flowering. The more pronounced the ruffle phenotype is present, i.e. the ratio of *w* to *l* being less, said phenotype tends to be maintained in time, i.e. also in successive flowerings, i.e. resulting in a stable ruffle phenotype. This is interesting for the grower who is interested to sell subsequent flowerings from the same mother plant. It would be undesirable when the ruffle phenotype would become lost or less pronounced in later flowerings. Nevertheless, the phenomenon of stable ruffle phenotype is unexpected, as the ruffle phenotype originates from a garden rose, which is not cut, and where no new stems develop induced by such cutting.

Selected offspring can also be further crossed to introduce desired characteristics, e.g. with regard to colour, foliage etc.

The invention therefor relates to a cut ruffle rose, of which the inventors were the first to succeed to develop. To this end the invention relates to a cut rose, comprising at least one flower comprising a plurality of petals, each petal being connected to the stem at the basal spot and having a longitudinal axis departing radially from the basal spot, the circumference of at least one of the said petals describing at least two recesses at opposite sides of the longitudinal axis, said recesses being directed towards the interior of the petal over a length *l*, therewith defining a tongue shaped petal portion therebetween, extending along the longitudinal axis, and a width w defined by the distance between the said recesses measured perpendicular to the longitudinal axis, said width w being equal to or less than length *l*. Hitherto, it was not possible to develop such a rose. It is believed that the improved germination results in sufficient offspring to enable offspring of the ruffle type to develop.

In an attractive embodiment, the circumference of at least 10%, preferably at least 20%, more preferably at least 30%, even more preferably at least 40% and most preferably at least 50% of the said petals describes the recesses as described above.

Very attractively, the ratio of the length *l* to width *w* in the roses of the invention is 2:1 to 10:1. Such plant can be obtained by selecting for such petal form. As described earlier, the more pronounced the ruffle phenotype manifests, the more stable the said phenotype will be maintained over multiple rounds of flowering, which is interesting for the grower who is interested to sell subsequent flowering from the same mother plant.

In an embodiment of the invention, the stem of the cut ruffle rose of the invention comprises a plurality of flowers, whereas in another embodiment the stem comprises a single flower. As described above, as the second parental line, and preferably also the first parental line is of the polyantha type, the offspring will produce a one or more cut ruffle roses that may still contain the polyantha phenotype. However, e.g. by crossing with a single flower type of rose, the polyantha phenotype can be removed, resulting in a cut ruffle rose of the single flower type. Such a rose is particularly attractive, as it resembles the dahlia, a very delicate cut flower, and only available during a very short period in the season. So the cut ruffle rose of the invention can also be regarded as a valuable replacement or alternative for the dahlia, which alternative is available over the year and being lass delicate.

Very surprisingly, it was found in the green house that the cut ruffle rose of the invention has an improved tolerance for thrips as compared to rose flowers, derived from a parental cut rose plant. Although the ruffle phenotype results in a more open architecture of the rose, it shows nevertheless improved tolerance for this plague. It would have been expected that thrips would have a more easy access to the ruffle rose as compared to the roses of a parental cut rose plant resulting in an increased vulnerability, which is surprisingly not the case. Thrips that are troublesome for cut roses are e.g. *Frankliniella occidentalis, Thrips fuscipennis* and *Echinothrips americanus*).

The invention also provides a rose plant capable of providing cut roses according to any of the preceding claims, or offspring thereof, preferably being capable of multiple flowering over the year, each flowering providing flowers of the ruffle type, i.e. having petals as defined above as well as a rose plant or offspring thereof, obtainable by the above described method.

The invention will now be further described by way of the following figures and examples.
Figure 1A is a schematic drawing of a petal of a 'normal' rose flower and figure 1B of the ruffle phenotype.
Figure 2 is a photograph of a normal cut rose with two petals removed.
Figure 3 is a photograph of a cut rose having a commencing but less pronounced ruffle phenotype with two petals removed.
Figure 4 is a photograph of a cut rose having ruffle phenotype with two petals removed.
Figure 5 is a photograph of a cut rose having ruffle phenotype with two petals removed, being more pronounced than the ruffle rose of figure 4.
Figure 6 is a photograph of another cut rose having ruffle phenotype with one petal removed.
Figures 7 - 22 are roses described in the examples.

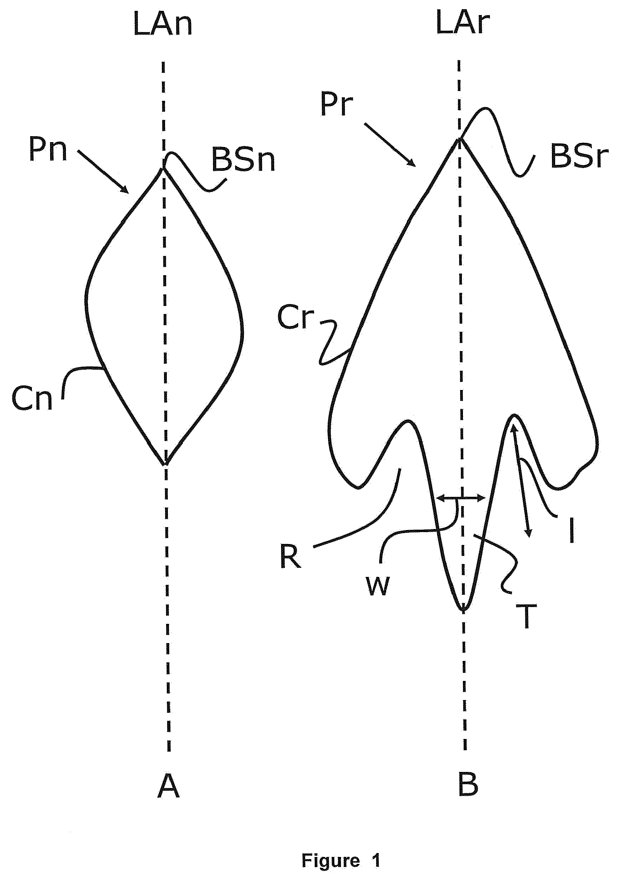

In figure 1A shows a normal rose petal Pn with circumference Cn and basal spot BSn, where Pn is connected to the stem (not shown). Vertical dotted line Lan is the longitudinal axis extending radially from the basal spot to the opposite end of the petal.

In figure 1B Petal Pr of the ruffled type is shown, with circumference Cr, and longitudinal axis Lar, extending from the basal spot BSr. Circumference Cr describes two recesses R at opposite sides of the longitudinal axis LAr, said recesses extending extending inwardly, directed to the interior of the petal over a length *l*, indicated with arrow I. Said recesses define a tongue shaped element T therebetween having a width w, said width being defined by the distance between said recesses measured perpendicular to the longitudinal axis Lar, said width being indicated by horizontal arrow w.

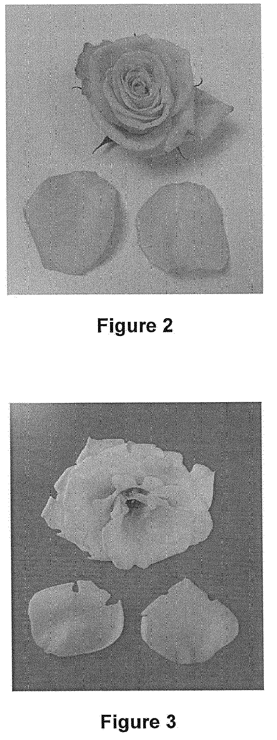

Figure 2 shows a normal cut rose flower 'Moonwalk' and two petals separated therefrom, said petals corresponding with the schematic drawing of figure 1.

Figure 3 shows a cut rose flower obtained by the method as described herein i.e. by crossing of a first and second parental line, showing a commencing ruffle phenotype, i.e. small inwardly extending recesses. The width between the two recessed is however larger than the length of the recesses.

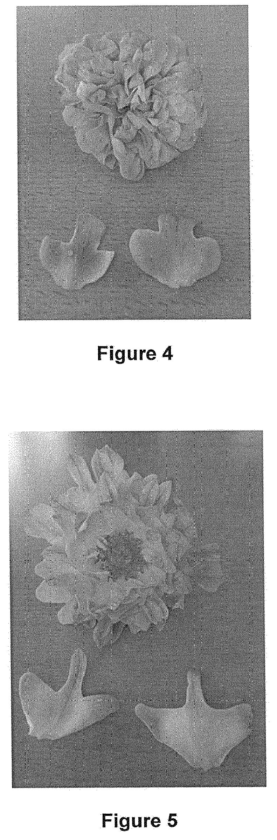

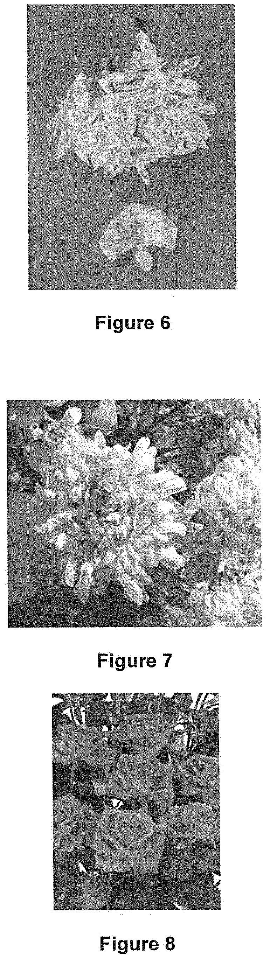

Figure 4 shows a cut ruffle rose flower and a petal separated therefrom, said petal corresponding with the schematic drawing of figure 1, where width w about equals length *l*. Figures 5 and 6 show cut ruffle rose flowers of a more pronounced ruffle type, i.e. where the *w* to *l* ratio is smaller as compared to that of the cut ruffle rose of figure 4.

### Example 1

### Development of cut ruffle roses of polyantha type

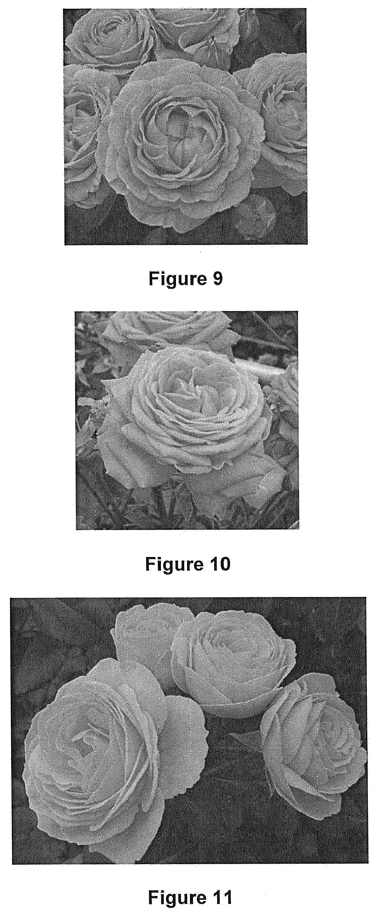

In a first crossing, a garden rose of the ruffle and floribunda type, B0187-04, see figure 7) was taken as first parental line and crossed with a cut spray rose Springtime (T0260-99, Interspritro, NL 20817) having normal petal shape as second parental line (see figure 8). Among the numerous offspring F1, a selection was made on petal form, and those offspring showing recesses on both sides of the longitudinal axis were selected for further breeding. Offspring Peche Mignon (T0127-07, Interjafume, JAP 21912, see figure 9) selected this way had cut flower properties, polyantha and commencing ruffle phenotype. Additional selection criteria of which one or more could also be taken into account when selecting offspring were a minimum number of 20 petals, more than one bud per stem, strong peduncle, long main stem, petal structure. Similar crossings were performed with Glamorous Ruffles, Red Lady Ruffles, Indian Ruffles and Ruffles Queen as first parental line, all garden roses of the ruffle and floribunda type, and resulted in similar offspring.

T0127-07 was crossed with Barbados (T1261-04, Interempros, NL 30653, figure 10), a cut spray rose with normal petal form, resulting in numerous F2 offspring, wherein a selection was made for ruffle phenotype, such as T0080-10 (figure 11).

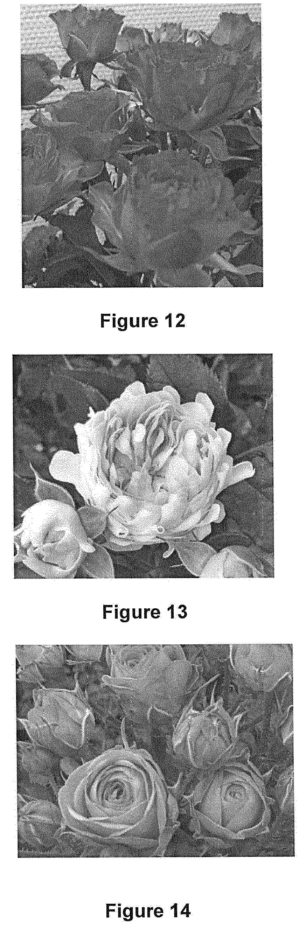

T0080-10 was crossed with T0525-10 (figure 12) another spray cut rose with normal petal form, resulting in a plurality of offspring having pronounced and stable ruffle phenotype, such as T0629-15 (figure 13).

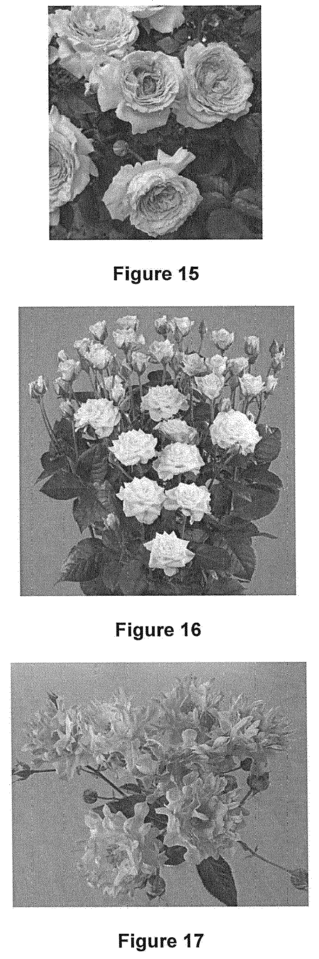

T0127-07 was crossed with T0669-05 (figure 14), a cut spray rose with normal petal form, resulting in offspring T2089-10, having a commencing ruffle phenotype, see figure 15. T2089-10 was crossed with Snowflake (T819-05, Intertrowit, EU 27987, figure 16), another spray cut rose with normal petal form, resulting in numerous offspring having pronounced and stable ruffle phenotype, such as T1090-12, see figure 17.

### Example 2

### Development of cut ruffle roses of the single flower type

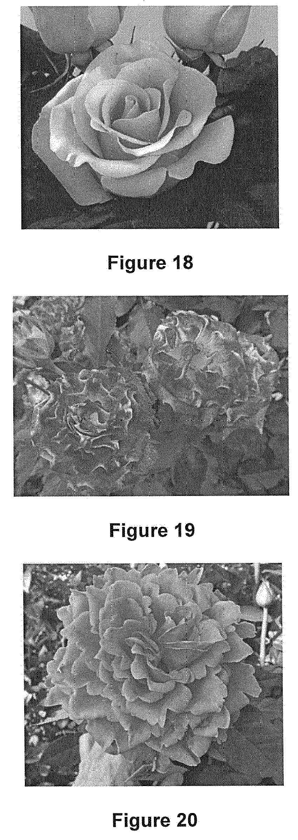

T1090-12 of example 1 was chosen for further breeding, and crossed with a cut single flower rose Titanic (Internaticti, JAP11811, EC 2010590, see figure 18), resulting in numerous offspring having commencing ruffled and polyantha phenotype, such as U0123-13 (figure 3) and U0762-13, (figure 19), respectively. Similar crossings were performed with Escimo, Circus, Iceberg or Akito, all cut single flower roses, with T1090-12, and resulted in similar offspring.

U0123-13 and U0762-13 were crossed with each other, resulting in numerous polyantha offspring of more pronounced ruffle phenotype, such as U0273-14, see figure 4, and U0489-14, see figure 5.

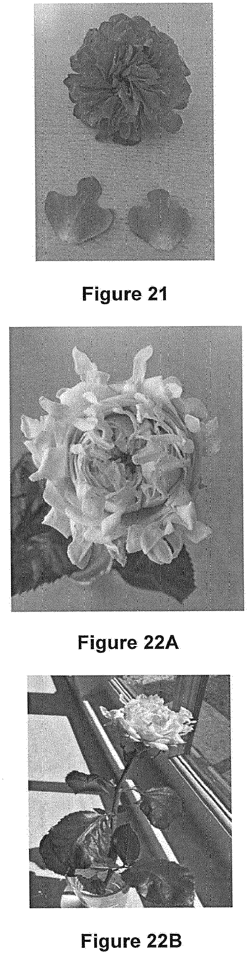

Both U0273-14 and U0489-14 were crossed with Titanic (see figure 18). From the numerous offspring having stable ruffle phenotype, two lines, U0168-15 (figure 20) and U1107-15 (figure 21), respectively, were crossed with one another, resulting in numerous cut single flower offspring having a stable ruffle phenotype, such as U374-16, see figures 22A and 22B.

## Claims

1. Cut rose, comprising at least one flower comprising a plurality of petals, each petal being connected to the stem at the basal spot and having a longitudinal axis departing radially from the basal spot, the circumference of at least one of the said petals describing at least two recesses at opposite sides of the longitudinal axis, said recesses being directed towards the interior of the petal over a length *l*, therewith defining a tongue shaped petal portion therebetween, extending along the longitudinal axis, and a width w defined by the distance between the said recesses measured perpendicular to the longitudinal axis, said width w being equal to or less than length *l*.

2. Cut rose of claim 1, wherein the circumference of at least 10%, preferably at least 20%, more preferably at least 30%, even more preferably at least 40% and most preferably at least 50% of the said petals describes at least two recesses at opposite sides of the longitudinal axis, said recesses being directed towards the interior of the petal over a length *l*, therewith defining a tongue shaped petal portion therebetween, extending along the longitudinal axis, and a width defined by the distance between the said recesses measured perpendicular to the longitudinal axis, said width being less than length *l*.

3. Cut rose of claim 1 or 2, wherein the ratio of the length *l* to width *w* is 2:1 to 10:1.

4. Cut rose of any of the preceding claims, wherein the stem comprises a plurality of flowers.

5. Cut rose of any of the preceding claims 1 - 3, wherein the stem comprises a single flower.

6. Cut rose of any of the preceding claims having improved tolerance for thrips as compared to rose flowers, derived from a parental cut rose plant.

7. Rose plant, capable of providing cut roses according to any of the preceding claims, or offspring thereof.

8. Rose plant or offspring thereof of claim 7, capable of multiple flowering over the year, each flowering providing flowers having petals as defined in any of the claims 1 - 3.

9. Rose plant or offspring thereof of any of claims 7 - 9, obtainable by a method comprising one or more crossings with a plurality of parental lines, a first parental line being a garden rose comprising one or more flowers as defined in any of claims 1 - 3, and a second parental line being a cut flower rose capable of providing a plurality of flowers per stem.

10. Rose plant or offspring thereof of claim 9, the first parental line having the floribunda phenotype.

11. Rose plant or offspring thereof of claim 9 or 10, the second parental line having more than 4 flowers per stem, preferably more than 10.

12. Rose plant or offspring thereof of any of the claims 9 - 11, the method comprising crossing of the first parental line with the second parental line.

13. Rose plant or offspring thereof of any of the claims 9 - 12, the method further comprising, after crossing with the first and the second parental line, a crossing with a third parental line, the third parental line being a cut flower rose providing a single flower per stem.

14. Rose plant or offspring thereof of any of claims 9 - 13, the method comprising the step of selecting offspring from one or more crossings on the presence of one or more flowers having one or more petals, the circumference thereof describing at least two recesses at opposite sides of the longitudinal axis, said recesses being directed towards the interior of the petal.

15. Rose plant or offspring thereof of claim 14, the method comprising the step of selecting offspring from one or more crossings on the presence of one or more flowers comprising a plurality of petals, each petal being connected to the stem at the basal spot and having a longitudinal axis departing radially from the basal spot, the circumference of at least one, preferably at least 10%, more preferably at least 20%, more preferably at least 30%, even more preferably at least 40% and most preferably at least 50% of the said petals describing at least two recesses at opposite sides of the longitudinal axis, said recesses being directed towards the interior of the petal over a length *l*, therewith defining a tongue shaped petal portion therebetween, extending along the longitudinal axis, and a width w defined by the distance between the said recesses measured perpendicular to the longitudinal axis, said width w being equal to or less than length.

## Amended claims

### Amended claims in accordance with Rule 137(2) EPC.

1. Cut rose, comprising at least one flower comprising a plurality of petals, each petal being connected to the stem at the basal spot and having a longitudinal axis departing radially from the basal spot, the circumference of at least one of the said petals describing at least two recesses at opposite sides of the longitudinal axis, said recesses being directed towards the interior of the petal over a length /, therewith defining a tongue shaped petal portion therebetween, extending along the longitudinal axis, and a width w defined by the distance between the said recesses measured perpendicular to the longitudinal axis, said width w being equal to or less than length /.

2. Cut rose of claim 1, wherein the circumference of at least 10%, preferably at least 20%, more preferably at least 30%, even more preferably at least 40% and most preferably at least 50% of the said petals describes at least two recesses at opposite sides of the longitudinal axis, said recesses being directed towards the interior of the petal over a length /, therewith defining a tongue shaped petal portion therebetween, extending along the longitudinal axis, and a width defined by the distance between the said recesses measured perpendicular to the longitudinal axis, said width being less than length *l*.

3. Cut rose of claim 1 or 2, wherein the ratio of the length *l* to width *w* is 2:1 to 10:1.

4. Cut rose of any of the preceding claims, wherein the stem comprises a plurality of flowers.

5. Cut rose of any of the preceding claims 1 - 3, wherein the stem comprises a single flower.

6. Cut rose of any of the preceding claims having improved tolerance for thrips as compared to rose flowers, derived from a parental cut rose plant.

7. Rose plant, capable of providing cut roses according to any of the preceding claims, or offspring thereof.

8. Rose plant or offspring thereof of claim 7, capable of multiple flowering over the year, each flowering providing flowers having petals as defined in any of the claims 1 - 3.

9. Rose plant or offspring thereof of any of claims 7 - 9, obtainable by a method comprising one or more crossings with a plurality of parental lines, a first parental line being a garden rose comprising one or more flowers as defined in any of claims 1 - 3, and a second parental line being a cut flower rose capable of providing a plurality of flowers per stem.

10. Rose plant or offspring thereof of claim 9, the first parental line having the floribunda phenotype.

11. Rose plant or offspring thereof of claim 9 or 10, the second parental line having more than 4 flowers per stem, preferably more than 10.

12. Rose plant or offspring thereof of any of the claims 9 - 11, the method comprising crossing of the first parental line with the second parental line.

13. Rose plant or offspring thereof of any of the claims 9 - 12, the method further comprising, after crossing with the first and the second parental line, a crossing with a third parental line, the third parental line being a cut flower rose providing a single flower per stem.

14. Rose plant or offspring thereof of any of claims 9 - 13, the method comprising the step of selecting offspring from one or more crossings on the presence of one or more flowers having one or more petals, the circumference thereof describing at least two recesses at opposite sides of the longitudinal axis, said recesses being directed towards the interior of the petal.

15. Rose plant or offspring thereof of claim 14, the method comprising the step of selecting offspring from one or more crossings on the presence of one or more flowers comprising a plurality of petals, each petal being connected to the stem at the basal spot and having a longitudinal axis departing radially from the basal spot, the circumference of at least one, preferably at least 10%, more preferably at least 20%, more preferably at least 30%, even more preferably at least 40% and most preferably at least 50% of the said petals describing at least two recesses at opposite sides of the longitudinal axis, said recesses being directed towards the interior of the petal over a length *l*, therewith defining a tongue shaped petal portion therebetween, extending along the longitudinal axis, and a width w defined by the distance between the said recesses measured perpendicular to the longitudinal axis, said width w being equal to or less than length *l*.
